# EUROPEAN PATENT APPLICATION

(11) **EP 4 578 309 A1**
(43) Date of publication of application: **02.07.2025**
(21) Application number: 23856219.3
(22) Date of filing: 16.06.2023
(51) Int. Cl.: A24F 40/465, A24F 40/57, H05B 6/02, H05B 6/06, H05B 6/80, A61M 15/00, A61M 11/00

(54) **AEROSOL GENERATING DEVICE AND AEROSOL GENERATING ARTICLE THEREOF, AND HEATING ASSEMBLY AND SUSCEPTOR**

(30) Priority: 26.08.2022 CN 202211035361
(71) Applicant: Shenzhen Merit Technology Co., Ltd., Shenzhen, Guangdong 518105 (CN)
(72) Inventor: HUANG, Zufu, Shenzhen, Guangdong 518105 (CN); LIANG, Feng, Shenzhen, Guangdong 518105 (CN); LI, Yu, Shenzhen, Guangdong 518105 (CN); HU, Guoqin, Shenzhen, Guangdong 518105 (CN)
(74) Representative: Westphal, Mussgnug & Partner, Patentanwälte mbB
(86) International application number: PCT/CN2023/100875
(87) International publication number: WO 2024/041127

(57) **Abstract**

The present invention relates to an aerosol generating device and an aerosol generating article thereof, and a heating assembly and a susceptor. The susceptor comprises a heating layer, a heat conduction layer and a temperature sensing layer, wherein the heating layer is used for generating heat based on the action of a changing magnetic field; the heat conduction layer is arranged between the heating layer and the temperature sensing layer, and the heat conduction layer is used for conducting to the temperature sensing layer the heat generated by the heating layer; and the temperature sensing layer is used for changing a magnetic permeability on the basis of the temperature of the susceptor. The heating assembly comprises a susceptor and a coil surrounding the susceptor. The aerosol generating article comprises an aerosol generating substrate and a susceptor. The aerosol generating device comprises a power source assembly and a heating assembly or an aerosol generating article. In the present invention, a magnetic permeability of a temperature sensing layer changes on the basis of the temperature of a susceptor, a heat conduction layer is added between the temperature sensing layer and a heating layer, different materials are respectively compounded to form the susceptor with a multilayer structure, and the structure of the susceptor is simple, thereby simplifying a temperature measurement of the susceptor; and the corrosion resistance of the materials is strong, thereby facilitating the accurate temperature control of the susceptor, and improving the heating efficiency.

## Description

### FIELD

The invention relates to the field of atomization, in particular to an aerosol generating device, an aerosol generating article, a heating assembly and a susceptor thereof.

### BACKGROUND

Currently, the main heating methods for heat-not-burn devices in the market are resistive heating and electromagnetic heating, among which the arrangement of heating elements in electromagnetic heating appliances is more flexible than resistive heating. No matter whether it is needle-type, chip-type, or circle, it is necessary to carry a temperature measuring wire for temperature feedback, to control the temperature variation range of the heating elements accurately, but this will reduce the application flexibility of the heating elements.

For electromagnetic heating, needle-type or chip-type or circumferential heating element is used. Traditionally, a temperature measuring film is printed on the outer surface and then connected to PCB through leads, so that the temperature of the heating element can be reflected through the feedback of the temperature measuring film. This way essentially reflects the temperature parameters of the heating element indirectly by borrowing the characteristics of other substances, which is mixed with many uncertainties, such as the heat conduction between the temperature measuring film and the heating element delays the heat exchange, and the temperature measuring film itself has heat capacity. A part of the energy is naturally consumed in the transmission process. At the same time, the process forming mode and protection mode of the temperature measuring film itself will limit the manufacturing shape of the heating element. Given these factors, the fixing of the heating element will become more complicated, which increases the structural cost and the fixing structure absorbs some energy of the heating element, reducing the efficiency of the heating element.

### SUMMARY

The technical problem to be solved by the present invention is to provide an aerosol generating device, an aerosol generating article, a heating assembly, and a susceptor thereof with a simple structure and high heating efficiency.

The technical scheme adopted by the invention to solve the technical problem is as follows: constructing a susceptor used in an aerosol generating device, comprising a heating layer, a heat conduction layer, and a temperature sensing layer, wherein the heating layer is used for generating heat based on the action of a changing magnetic field; the heat conduction layer is arranged between the heating layer and the temperature sensing layer; the heat conduction layer is used for conducting the heat generated by the heating layer to the temperature sensing layer; and the temperature sensing layer is used for changing a magnetic permeability based on the temperature of the susceptor.

Preferably, the magnetic permeability of the temperature sensing layer changes based on the temperature change within a preset temperature range.

Preferably, the preset temperature range is greater than or equal to a first temperature value and less than or equal to a second temperature value, and the second temperature value is lower than the Curie point temperature of the material of the temperature sensing layer.

Preferably, within the preset temperature range, the magnetic permeability of the temperature sensing layer gradually increases with the gradual increase of temperature.

Preferably, within the preset temperature range, the magnetic permeability of the temperature sensing layer gradually decreases with the gradual increase of temperature.

Preferably, the material of the temperature sensing layer comprises a soft magnetic material.

Preferably, the material of the temperature sensing layer includes 1j85 material.

Preferably, the Curie point temperature of the material of the temperature sensing layer is lower than 800 °C.

Preferably, the material of the heating layer comprises ferromagnetic material, and the Curie point temperature of the material of the heating layer is higher than the Curie point temperature of the material of the temperature sensing layer.

Preferably, the material of the heating layer comprises a nonmagnetic material.

Preferably, the material of the heat conduction layer includes a diamagnetic material.

Preferably, the material of the heat conduction layer includes any one or more of gold, silver, copper, and graphene.

Preferably, the shape of the susceptor is blade-shaped, and the susceptor comprises a sheet-shaped first body part and a first sharp end connected to the top end of the first body part.

Preferably, the heating layer, the heat conduction layer, and the temperature sensing layer are parallel to each other, and the three are stacked on each other.

Preferably, the thickness ratio of the heating layer, the heat conduction layer, and the temperature sensing layer is 5:1:4.

Preferably, the susceptor is tubular in shape.

Preferably, the susceptor has a cylindrical shape, and the susceptor comprises a cylindrical second body part and a second sharp end connected to the top end of the second body part.

Preferably, the heat conduction layer and the temperature sensing layer are at least partially embedded in the heating layer, and the heat conduction layer separates the temperature sensing layer from the heating layer.

Preferably, the thickness ratio of the heating layer, the heat conduction layer, and the temperature sensing layer is 6:1:3.

Preferably, the number of the heat conduction layers is two or more.

Preferably, the number of the temperature sensing layers is two or more.

The invention also constructs a heating assembly, comprising the susceptor and a coil surrounding the susceptor for generating a magnetic field.

The invention also constructs an aerosol generating article, comprising an aerosol generating substrate and a susceptor, wherein the susceptor is used for heating the aerosol generating substrate.

The invention also constructs an aerosol generating device, comprising a power source assembly and the heating assembly or the aerosol generating article; the power source assembly is used for driving the susceptor to generate heat.

The invention has the following beneficial effects: the temperature sensing layer of the invention changes the magnetic permeability based on the temperature of the susceptor, and a heat conduction layer with high thermal conductivity is added between the temperature sensing layer and the heating layer, and different materials are respectively compounded to form the susceptor with a multilayer structure so that the structure is simple, the temperature measurement of the susceptor is simplified, and the corrosion resistance is strong, which is beneficial to realize accurate temperature control of the susceptor and improve heating efficiency.

### BRIEF DESCRIPTION OF THE DRAWINGS

The invention will be further described with reference to the attached drawings and examples, in which:
Figure 1 is a cross-sectional view of an embodiment of the susceptor of the present invention;
Figure 2 is a cross-sectional view of another embodiment of the susceptor of the present invention;
Figure 3 is a cross-sectional view of other embodiments of the susceptor of the present invention;
Figure 4 is a schematic diagram of the change of magnetic permeability and temperature of the material of the susceptor of the present invention;
Figure 5 is a schematic structural view of the first embodiment of the susceptor of the present invention;
Figure 6 is an exploded view of the structure of the susceptor of Figure 5;
Figure 7 is a schematic structural view of a second embodiment of the susceptor of the present invention;
Figure 8 is a schematic structural view of another embodiment of Figure 7;
Figure 9 is a schematic structural view of a third embodiment of the susceptor of the present invention; and
Figure 10 is a schematic structural view of another embodiment of Figure 9.

### DETAILED DESCRIPTION

To have a clearer understanding of the technical characteristics, purpose, and effect of the invention, the specific embodiments of the invention are described in detail against the attached drawings. In the following description, it should be understood that the orientation or position relationship indicated by "front", "rear", "upper", "lower", "left", "right", "longitudinal", "transverse", "vertical", "horizontal", "top", "bottom", "inside", "outside", "head", "tail", etc., is based on the orientation or position relationship shown in the attached figure, and is constructed and operated in a specific orientation. It is intended only to facilitate the description of the technical scheme, not to indicate that the device or element referred to must have a specific orientation, and therefore cannot be understood as a limitation of the invention.

It should also be noted that unless otherwise specified and limited, terms such as "installation", "connection", "link", "fixing" and "setting" should be understood broadly, for example, they can be fixed connection, detachable connection or integrated; can be a mechanical connection or an electrical connection; can be directly connected, can also be indirectly connected through an intermediary, can be the internal connection of two elements or the interaction between two elements. When an element is said to be "above" or "below" another element, the element can be "directly" or "indirectly" above the other element, or there may be one or more intervening elements. The terms "first", "second" and "third" are only for the convenience of describing the technical scheme, and cannot be understood as indicating or implying relative importance or implicitly indicating the number of indicated technical features. Therefore, features defined as "first", "second" and "third" can explicitly or implicitly include one or more such features. For those skilled in the art, the specific meanings of the above terms in the present invention can be understood according to specific situations.

In the following description, specific details, such as specific system structure and technology, are set forth for the purpose of explanation rather than limitation, to thoroughly understand the embodiments of the present invention. However, it will be apparent to one skilled in the art that the present invention may be practiced in other embodiments without these specific details. In other cases, detailed descriptions of well-known systems, devices, circuits, and methods are omitted so as not to obscure the description of the present invention with unnecessary details.

It should be noted that the crux of the problem of electromagnetic heating in the present invention mainly lies in the susceptor itself.

The basic principle of electromagnetic heating is that the changing current forms a changing magnetic field through the electromagnetic coil, and the changing magnetic field acts on the susceptor, the susceptor body generates eddy current, the eddy current is converted into heat. The actual electromagnetic coil body has certain attribute parameters, such as inductance, AC resistance, linear resistance, quality factor and other parameters. When the susceptor is placed in the influence range of the electromagnetic coil, these parameters will change with the position, size, material, volume in the influence zone and temperature of the susceptor in the influence zone except the linear resistance.

The magnetic properties of the electromagnetic heating susceptor material directly affect the heating temperature rise rate and efficiency of the susceptor. For metals with good magnetic properties, the susceptor material in the invention belongs to soft magnetic materials, and the important characteristic parameter for describing soft magnetic materials is magnetic permeability. It is found that the change of characteristic parameters of electromagnetic coils is directly related to the magnetic permeability of the susceptor.

For general susceptors, the important factor that restricts their flexible application and configuration is the need for external temperature measurement to assist, while for the magnetic permeability of some soft magnetic materials changes with the temperature change of the body, so that the temperature change being transformed into the magnetic permeability change can be established, and then the magnetic permeability change is transformed into the electrical parameter change of the coil itself, and further, the temperature change is transformed into the electrical parameter change of the coil itself. Only the change of electrical parameters of the coil needs to be measured, and the temperature value of heating can be calculated from the measured value, thus forming a complete temperature feedback link, through the link the temperature of the susceptor can be controlled.

For soft magnetic materials within the temperature control range, stainless steel such as 430 not only has higher heating efficiency but also has strong corrosion resistance and simple surface protection. Based on the advantages that these two can achieve different goals, it is considered to compound the two materials to form a susceptor for temperature measurement. Because the two materials are compounded together, their thermal conductivity is lower than the thermal conductivity of precious metals such as copper and silver, and the temperature rising speed of the soft magnetic materials as temperature sensing is not as fast as the temperature rising speed of 430/410 stainless steel. If it is necessary to characterize the overall temperature of the composite susceptor, the temperature-sensing material needs to absorb the heat of the heating material, and at the same time, the temperature-sensing material can better feedback on the temperature change after the overall heat soaking. Based on this, a layer of high thermal conductivity material is added between the temperature-sensing material and the heating material, and the invention can realize simplified temperature measurement.

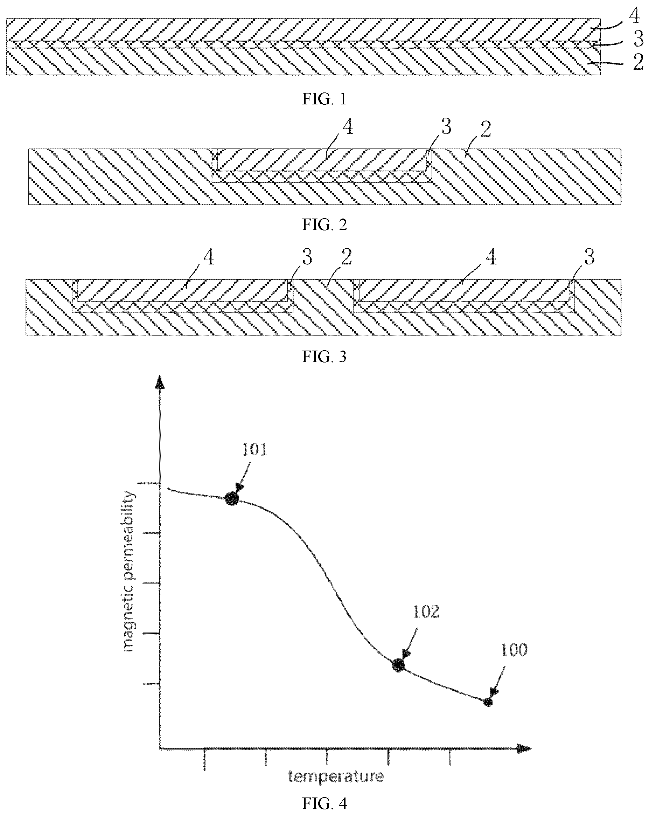

Figure 1 shows susceptor 1 of the present invention, which is used in an aerosol generating device. The susceptor 1 includes three layers, including a heating layer 2, a heat conduction layer 3, and a temperature sensing layer 4 respectively. The heating layer 2 is used for generating heat based on the action of a changing magnetic field, and the heat conduction layer 3 is arranged between the heating layer 2 and the temperature sensing layer 4, and the heating layer 2 is in contact with the heat conduction layer 3, the heat conduction layer 3 is used for conducting the heat generated by the heating layer 2 to the temperature sensing layer 4, and the temperature sensing layer 4 is used for changing a magnetic permeability on the basis of the temperature of the susceptor 1.

As shown in Figure 1, in some embodiments, the susceptor 1 has a three-layer superimposed structure. Specifically, the heating layer 2, the heat conduction layer 3, and the temperature sensing layer 4 are parallel to each other and are laminated with each other, wherein the dimensions of the heating layer 2, the heat conduction layer 3, and the temperature sensing layer 4 can be approximately the same to facilitate mutual lamination. As shown in Figure 2, in other embodiments, the susceptor 1 has a three-layer nested structure, in which the heating layer 2, the heat conduction layer 3, and the temperature sensing layer 4 are nested with each other, and the heating layer 2 is used as the base material, and the heat conduction layer 3 is embedded in part of the heating layer 2, while the temperature sensing layer 4 is embedded in the heat conduction layer 3, the heat conduction layer 3 completely separates the temperature sensing layer 4 from the heat sensing layer 2. That is to say, the heating layer 2 is provided with a first slot, and the heat conduction layer 3 is embedded in the first slot of the heating layer 2. At the same time, the temperature sensing layer 4 is provided with a second slot, and the temperature sensing layer 4 is embedded in the second slot of the heat conduction layer 3. The diameter of the first slot is larger than the diameter of the second slot, and finally the heat conduction layer 3 separates the temperature sensing layer 4 from the heating layer 2.

In some embodiments, the heating layer 2, the heat conduction layer 3, and the temperature sensing layer 4 can also have other forms of composite structures. The temperature sensing layer 4 can be arranged in the middle, and the heat conduction layer 3 is arranged outside the temperature sensing layer 4, and the temperature sensing layer 4 can be completely wrapped by the heat conduction layer 3; the heating layer 2 is arranged outside the heat conduction layer 3, and the heat conduction layer 3 can be completely wrapped by the heating layer 2. In other embodiments, the size of the heat conduction layer 3 may be different from the size of the temperature sensing layer 4, that is, the heat conduction layer 3 may only partially cover the surface of the side contacting with the temperature sensing layer 4.

In some embodiments, the number of temperature sensing layers 4 of the susceptor can be two or more, and the number of heat conduction layers 3 can also be two or more, which is not limited here. As shown in Figure 3, the susceptor includes two temperature sensing layers 4, two heat conduction layers 3, and a heating layer 2, wherein one temperature sensing layer 4 is wrapped by one heat conduction layer 3 and arranged on one side of the heating layer 2, and the other temperature sensing layer 4 is wrapped by another heat conduction layer 3 and arranged on the other side of the heating layer 2, and a certain distance can be formed between two adjacent temperature sensing layers 4.

Within the preset temperature range, the magnetic permeability of the temperature sensing layer 4 changes with the change of temperature. By establishing the relationship between the temperature change characteristics and the magnetic permeability change of the same material, the relationship between them can be linear or nonlinear. The preset temperature range may be greater than or equal to the first temperature value and less than or equal to the second temperature value, and the second temperature value is lower than the Curie point temperature of the material of the temperature sensing layer 4. Within the preset temperature range, the magnetic permeability of the temperature sensing layer 4 changes with the change of temperature, and each of magnetic permeability can have a corresponding temperature value, so the temperature change can be obtained according to the change of magnetic permeability.

In some embodiments, within a preset temperature range, the magnetic permeability of the temperature sensing layer 4 may gradually decrease with the gradual increase of temperature; in other embodiments, within the preset temperature range, the magnetic permeability of the temperature sensing layer 4 may gradually increase with the gradual increase of temperature.

In some embodiments, the material of the temperature sensing layer 4 can be functional soft magnetic materials, all of which have a Curie point temperature, while the application range of the aerosol generating substrate is within 500°C. Through the study of different soft magnetic materials, it is found that the magnetic permeability of some materials does not change obviously with temperature below Curie point, but only when they are close to Curie point, it is obvious with the change of temperature. However, the magnetic permeability of some materials gradually decreases with the increase of temperature below Curie point temperature, and this change of parameter characteristics is exactly what the invention needs in practical application. Generally, the Curie point 100 temperature of the material of the temperature sensing layer 4 is lower than 800°C, and the Curie point 100 temperature of the material of the temperature sensing layer 4 is preferably around 400°C. As shown in Figure 4, specifically, the material magnetic permeability of the temperature sensing layer 4 has a first temperature change point 101 and a second temperature change point 102, wherein the temperature of the first temperature change point 101 and the second temperature change point 102 are both lower than the Curie point 100 temperature of the material of the temperature sensing layer 4. The temperature corresponding to the first temperature change point 101 can be selected as the first temperature value, and the temperature corresponding to the second temperature change point 102 can be selected as the second temperature value. Before the temperature of the soft magnetic material of the temperature sensing layer 4 reaches the first temperature change point 101, the magnetic permeability of the soft magnetic material of the temperature sensing layer 4 is basically unchanged. When the temperature of the soft magnetic material of the temperature sensing layer 4 reaches the first temperature change point 101 and the temperature is constantly rising, the magnetic permeability of the soft magnetic material of the temperature sensing layer 4 begins to gradually and regularly decrease with the temperature increase until the temperature rises to the second temperature change point 102. After the temperature rises to the second temperature change point 102, the magnetic permeability of the soft magnetic material of the temperature sensing layer 4 begins to reduce the change rate until the temperature reaches Curie point 100 of the soft magnetic material of the temperature sensing layer 4. When the temperature reaches Curie point 100 of the soft magnetic material of the temperature sensing layer 4, the magnetic permeability decreases to zero, and the material of the temperature sensing layer 4 completely loses its magnetism.

In some embodiments, the material of the heating layer 2 includes ferromagnetic material, such as iron or iron alloy, the iron alloy can be grade 410 stainless steel, grade 420 stainless steel or grade 430 stainless steel; wherein, grade 430 stainless steel is ferromagnetic material with Curie temperature exceeding 400°C. When the material of the heating layer 2 is ferromagnetic material, the Curie point temperature of the material of the heating layer 2 needs to be higher than the Curie point temperature of the material of the temperature sensing layer 4. Specifically, the material of the heating layer 2 can also be a non-magnetic material, such as aluminum.

In some embodiments, the thermal conductive layer 3 can be made of metal materials with high thermal conductivity, such as common metals such as gold, silver and copper. Non-metallic materials with higher thermal conductivity, such as graphene, can also be used; therefore, the material of the heat conduction layer 3 includes any one or more of gold, silver, copper and graphene. In some embodiments, the material of the heat conduction layer 3 includes a diamagnetic material so that the heat generated by the heat conduction layer 3 in the changing magnetic field is less, and the heat received by the temperature sensing layer 4 basically comes from the heat generated by the heating layer 2 conducted from the heat conduction layer 3.

The material of the temperature sensing layer 4 may include 1j85 material in some embodiments. The 1j85 material, also known as 1j85 permalloy, is a Nickel-Iron alloy with high magnetic permeability and low coercivity.

The shape of the susceptor 1 can be sheet, tube, column, etc., and is not limited here.

The invention also constructs a heating assembly, which includes the above-mentioned susceptor 1 and a coil for generating a magnetic field around the susceptor 1. In some embodiments, the heating assembly may further include a fixing base 5 provided at the lower part of the susceptor 1, and the fixing base 5 is used for fixing the susceptor 1. The invention also constructs an aerosol generating article, which includes an aerosol generating substrate and the above-mentioned susceptor 1, and the susceptor 1 is used for heating the aerosol generating substrate.

The invention also constructs an aerosol generating device, which includes the above-mentioned heating assembly or the above-mentioned aerosol generating article. The power source assembly is used to drive the susceptor to generate heat, and the power source assembly may include a battery. The aerosol generating device can comprise a housing, a heating assembly arranged in the housing and a battery arranged in the housing and electrically connected with two electrode leads of the heating assembly, and an aerosol generating substrate can be inserted into the housing from the top of the housing. The upper end of the heating assembly is inserted into the aerosol generating substrate, and the aerosol generating substrate is baked and heated after the heating assembly iselectrified to raise the temperature, to form an aerosol which can be sucked by users.

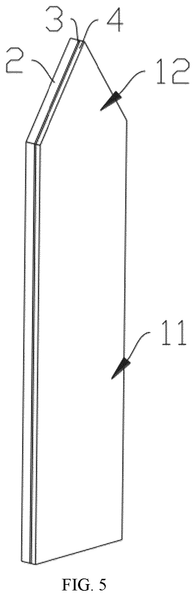

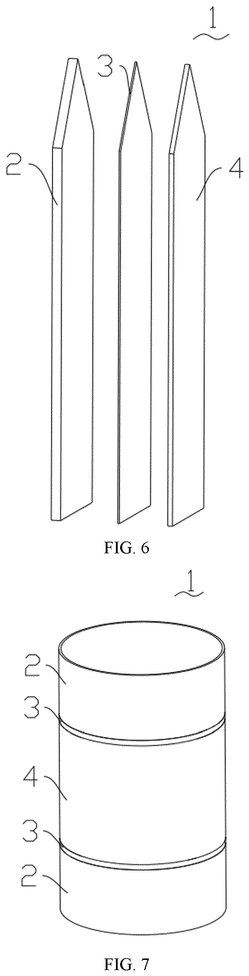

Figure 5 shows a susceptor 1 of a first embodiment of the present invention. In this embodiment, the susceptor 1 is in the shape of a blade, and comprises a sheet-like first body part 11 and a first sharp end 12 connected to the top end of the first body part 11, wherein the first body part 11 has a rectangular sheet-like structure and the first sharp end 12 has a triangular sheet-like structure, and the length of the bottom of the first sharp end 12 is the same as the length of the narrow side of the first body part 11, the first body part 11 and the first sharp end 12 may be integrally formed. As shown in Figure 6, in this embodiment, the susceptor 1 is composed of a three-layer superimposed structure, and the heating layer 2, the heat conduction layer 3, and the temperature sensing layer 4 are parallel to each other, and they are stacked with each other.

In this embodiment, the thickness ratio among the heating layer 2, the heat conduction layer 3, and the temperature sensing layer 4 can be 5: 1: 4. In one embodiment, the total thickness of the susceptor 1 is 0.5mm, in which the heating layer 2 accounts for 50%, the temperature sensing layer 4 accounts for 40%, and the heat conduction layer 3 accounts for 10%. After molding, the three layers are parallel to each other. Specifically, the heating layer 2 is made of grade 430 stainless steel, the temperature sensing layer 4 is made of 1j85 material, and the heat conduction layer 3 is made of copper.

According to the requirements of electromagnetic environment for the skin effect of the susceptor 1, the susceptor 1 can be made into a sheet-like part with a shape of 12mm × 4mm by using three layers of parallel composite materials pressed according to the above dimensions, one end of which is fixed on the fixing base 5 in the aerosol generating device, and the other end is symmetrically chamfered, so as to facilitate the insertion and guidance of the aerosol generating substrate. The Curie point of the temperature sensing layer 4 is about 400°C, and the susceptor 1 is placed in the induction center of the electromagnetic coil. After inserting the aerosol generating substrate, the coil is connected with high-frequency current. When the temperature of the susceptor 1 reaches the temperature of the first temperature change point 101, the temperature of the first temperature change point 101 is 150 degrees Celsius, and the magnetic permeability of the temperature sensing layer 4 gradually and regularly decreases, thus causing the electrical parameters of the coil to change accordingly; by capturing the electrical parameters and matching the data processing, the current temperature value of the susceptor 1 can be inferred. According to the characteristic of alternating current, the temperature value of the susceptor 1 can be detected in real time . Usually, when the aerosol substrate is heated, the target temperature of the susceptor 1 is about 300°C. Because this target temperature is less than the temperature of the second temperature change point 102 of the material, for example, the temperature of the second temperature change point 102 is 380°C, therefore, it can be ensured that the relationship between the temperature and the magnetic permeability of the susceptor 1 changes regularly during the process of heating the susceptor 1 from the first temperature change point 101 to the target temperature of 300°C, and the temperature corresponds to the magnetic permeability one by one. Therefore, the magnetic permeability of the material of the temperature sensing layer 4 changes regularly with the temperature, so that the temperature value corresponding to each of magnetic permeability in this temperature range can be achieved, and the temperature control accuracy can be achieved within 1°C.

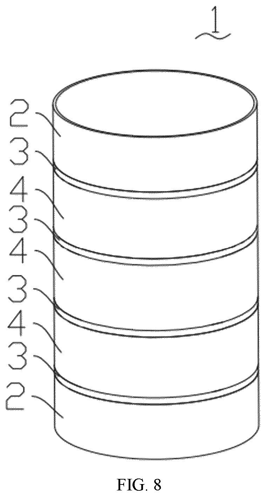

Figure 7 shows a susceptor 1 according to a second embodiment of the present invention, and the susceptor 1 may have a hollow tubular shape. In this embodiment, the susceptor 1 consists of a three-layer nested structure, that is, a heating layer 2, a heat conduction layer 3, and a temperature sensing layer 4 are nested with each other, and the heat conduction layer 3 and the temperature sensing layer 4 are at least partially embedded in the heating layer 2, and the heat conduction layer 3 separates the temperature sensing layer 4 from the heating layer 2. In some embodiments, the number of temperature sensing layers of the susceptor can be two or more, and the number of heat conduction layers can also be two or more, which is not limited here. As shown in Figure 8, the susceptor is made of three-layer nested composite material including two temperature sensing layers 4, two heat conduction layers 3, and a heating layer 2 as shown in Figure 3.

In this embodiment, the thickness ratio among the heating layer 2, the heat conduction layer 3, and the temperature sensing layer 4 can be 6:1:3. In one embodiment, the total thickness of the susceptor 1 is 0.5mm, in which the heating layer 2 accounts for 60%, the temperature sensing layer 4 accounts for 30%, and the heat conduction layer 3 accounts for 10%. Specifically, the heating layer 2 is made of grade 430 stainless steel, the temperature sensing layer 4 is made of 1j85 material, and the heat conduction layer 3 is made of copper.

According to the requirements of electromagnetic environment for skin effect of the susceptor 1, the susceptor 1 can be made into a tubular part with a diameter of 7mm and a length of 12mm by rolling three layers of nested composite materials pressed in the above manner, one end of which is fixed on the guide to facilitate the insertion and guidance of the aerosol generating substrate, and the Curie point of the material of the temperature sensing layer 4 is about 400°C. The susceptor 1 is placed in the induction center of the electromagnetic coil, and after the aerosol generating substrate is inserted, high-frequency current is applied to the coil. When the temperature of the susceptor 1 reaches the temperature of the first temperature change point 101, the temperature of the first temperature change point 101 is 150 degrees Celsius, and the magnetic permeability of the temperature sensing layer 4 gradually and regularly decreases, thus causing the electrical parameters of the coil to change. By capturing the electrical parameters by the circuit and performing data processing and matching, the current temperature value of the susceptor 1 can be inferred. According to the characteristic of alternating current, the temperature value of the susceptor 1 can be detected in real time. Usually, when the aerosol substrate is heated, the target temperature of the susceptor 1 is about 300°C. Because this target temperature is less than the temperature of the second temperature change point 102 of the material, for example, the temperature of the second temperature change point 102 is 380°C. Therefore, it can be ensured that the relationship between the temperature and the magnetic permeability of the susceptor 1 changes regularly during the process of heating the susceptor 1 from the first temperature change point 101 to the target temperature of 300°C, and the temperature corresponds to the magnetic permeability one by one. Therefore, the magnetic permeability of the temperature sensing layer 4 changes regularly with the temperature, and the temperature control accuracy is within 1°C.

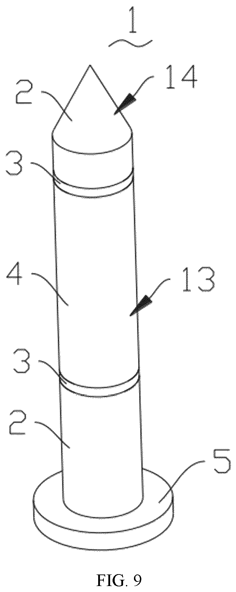

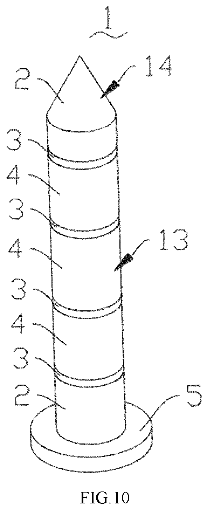

Figure 9 shows a susceptor 1 according to a third embodiment of the present invention. The susceptor 1 may be cylindrical in shape with a hollow interior, and is generally a needle-type susceptor 1. The susceptor 1 includes a cylindrical second body portion 13 and a second sharp end 14 connected to the top end of the second body portion 13. The second body part 13 has a cylindrical structure, and the second sharp end 14 has a conical structure. The bottom area of the second sharp end 14 is the same as the top area of the second body part 13, and the second body part 13 and the second sharp end 14 can be integrally formed. Understandably, the shape of the susceptor 1 can also be other shapes such as rectangular parallelepiped, rod, etc., and there is no limitation here. Specifically, in this embodiment, the susceptor 1 can be composed of a three-layer nested structure, wherein the heating layer 2, the heat conduction layer 3, and the temperature sensing layer 4 are nested with each other, the heat conduction layer 3 and the temperature sensing layer 4 are at least partially embedded in the heating layer 2, and the heat conduction layer 3 separates the temperature sensing layer 4 from the heating layer 2. In some embodiments, the number of temperature sensing layers of the susceptor can be two or more, and the number of heat conduction layers can also be two or more, which is not limited here. As shown in Figure 10, the susceptor is made of three-layer nested composite material including two temperature sensing layers 4, two heat conduction layers 3, and a heating layer 2 as shown in Figure 3.

In this embodiment, the thickness ratio among the heating layer 2, the heat conduction layer 3, and the temperature sensing layer 4 can be 6: 1: 3. In one embodiment, the total thickness of the susceptor 1 is 0.5mm, in which the heating layer 2 accounts for 60%, the temperature sensing layer 4 accounts for 30%, and the heat conduction layer 3 accounts for 10%. Specifically, the heating layer 2 is made of grade 430 stainless steel, the temperature sensing layer 4 is made of 1j85 material, and the heat conduction layer 3 is made of copper.

According to the design requirements of the needle-type susceptor, the susceptor 1 can be made into a needle-type part with a diameter of 2mm and a length of 12mm by using the three-layer nested composite material roll pressed in the above manner, one end of which is fixed on the fixing base 5 of the aerosol generating device, and the other end is chamfered, to facilitate the insertion and guidance of the aerosol generating substrate. The Curie point of this material is about 400°C. The susceptor 1 is placed in the induction center of the electromagnetic coil. After inserting the aerosol generating substrate, high-frequency current is applied to the coil. When the temperature of the susceptor 1 reaches the temperature of the first temperature change point 101, the temperature of the first temperature change point 101 is 150 degrees Celsius, the magnetic permeability of the temperature sensing layer 4 begins to decrease gradually and regularly, thus causing the electrical parameters of the coil to change. By capturing the electrical parameters and matching the data processing, the current susceptor can be inferred. According to the characteristic of alternating current, the temperature value of the susceptor 1 can be detected in real time. Usually, when the aerosol substrate is heated, the target temperature of the susceptor 1 is about 300°C. Because this target temperature is less than the temperature of the second temperature change point 102 of the material, for example, the temperature of the second temperature change point 102 is 380 °C. Therefore, it can be ensured that the relationship between the temperature and the magnetic permeability of the susceptor 1 changes regularly during the process of heating the susceptor 1 from the first temperature change point 101 to the target temperature of 300°C. Therefore, the magnetic permeability of the temperature sensing layer 4 changes regularly with the temperature, and the temperature control accuracy is within 1°C.

The susceptor of the present invention adds a layer of high thermal conductivity between the temperature sensing layer and the heating layer, and different materials are respectively compounded to form the susceptor with a multilayer structure, so that the soft magnetic material and the ferromagnetic material are combined together, and the structure is simple, and the temperature measurement of the susceptor is simplified; moreover, compared with the susceptor made of single-material soft magnetic functional materials, the manufacturing cost is saved. At the same time, compared with soft magnetic materials within the temperature control range, stainless steel such as 430 not only has higher heating efficiency, but also has strong corrosion resistance and simple surface protection, which is beneficial to realize accurate temperature control of the susceptor and improve heating efficiency.

It can be understood that the above embodiment only expresses the preferred embodiment of the present invention, and its description is more specific and detailed, but it cannot be understood as limiting the patent scope of the present invention; it should be pointed out that for ordinary technicians in this field, the above technical features can be freely combined without departing from the concept of the present invention, and several modifications and improvements can be made, which are all within the scope of protection of the present invention; therefore, all equivalent transformations and modifications made with the scope of the claims of the present invention should belong to the scope of the claims of the present invention.

## Claims

1. A susceptor used in an aerosol generating device, comprising a heating layer (2), a heat conduction layer (3), and a temperature sensing layer (4), wherein the heating layer (2) is used for generating heat based on the action of a changing magnetic field, the heat conduction layer (3) is arranged between the heating layer (2) and the temperature sensing layer (4), and the heat conduction layer(3) is used for conducting the heat generated by the heating layer(2) to the temperature sensing layer(4); and the temperature sensing layer(4) is used for changing a magnetic permeability on the basis of the temperature of the susceptor.

2. The susceptor according to claim 1, wherein the magnetic permeability of the temperature sensing layer (4) changes based on the change of temperature within a preset temperature range.

3. The susceptor according to claim 2, wherein the preset temperature range is greater than or equal to a first temperature value and less than or equal to a second temperature value, and the second temperature value is lower than the Curie point temperature of the material of the temperature sensing layer (4).

4. The susceptor according to claim 3, wherein within the preset temperature range, the magnetic permeability of the temperature sensing layer (4) gradually increases with the gradual increase of temperature.

5. The susceptor according to claim 3, wherein within the preset temperature range, the magnetic permeability of the temperature sensing layer (4) gradually decreases with the gradual increase of temperature.

6. The susceptor according to claim 2, wherein the material of the temperature sensing layer (4) comprises a soft magnetic material.

7. The susceptor according to claim 6, wherein the material of the temperature sensing layer (4) comprises 1j85 material.

8. The susceptor according to claim 1, wherein the Curie point temperature of the material of the temperature sensing layer (4) is lower than 800°C.

9. The susceptor according to claim 1, wherein the material of the heating layer (2) comprises a ferromagnetic material, and the Curie point temperature of the material of the heating layer (2) is higher than the Curie point temperature of the material of the temperature sensing layer (4).

10. The susceptor according to claim 1, wherein the material of the heating layer (2) comprises a nonmagnetic material.

11. The susceptor according to claim 1, wherein the material of the heat conduction layer (3) comprises a diamagnetic material.

12. The susceptor according to claim 11, wherein the material of the heat conduction layer (3) comprises any one or more of gold, silver, copper and graphene.

13. The susceptor according to claim 1, wherein the susceptor (1) is blade-shaped, and the susceptor (1) comprises a sheet-shaped first body part (11) and a first sharp end (12) connected to the top end of the first body part (11).

14. The susceptor according to claim 13, wherein the heating layer (2), the heat conduction layer (3), and the temperature sensing layer (4) are parallel to each other, and the three are stacked on each other.

15. The susceptor according to claim 14, wherein the thickness ratio of the heating layer (2), the heat conduction layer (3), and the temperature sensing layer (4) is 5:1:4.

16. The susceptor according to claim 1, wherein the susceptor (1) is tubular in shape.

17. The susceptor according to claim 1, wherein the susceptor (1) has a cylindrical shape, and the susceptor (1) comprises a cylindrical second body part (13) and a second sharp end (14) connected to the top end of the second body part (13).

18. The susceptor according to claim 16 or 17, wherein the heat conduction layer (3) and the temperature sensing layer (4) are at least partially embedded in the heating layer (2), and the heat conduction layer (3) separates the temperature sensing layer (4) from the heating layer (2).

19. The susceptor according to claim 18, wherein the thickness ratio of the heating layer (2), the heat conduction layer (3), and the temperature sensing layer (4) is 6:1:3.

20. The susceptor according to claim 18, wherein the number of the heat conduction layers (3) is two or more.

21. The susceptor according to claim 18, wherein the number of the temperature sensing layers (4) is two or more.

22. A heating assembly, comprising a susceptor (1) according to any one of claims 1 to 21 and a coil surrounding the susceptor (1) for generating a magnetic field.

23. An aerosol generating article, comprising an aerosol generating substrate and a susceptor (1) according to any one of claims 1 to 21, wherein the susceptor (1) is used for heating the aerosol generating substrate.

24. An aerosol generating device, comprising a power source assembly; and the heating assembly according to claim 22 or the aerosol generating article according to claim 23; the power source assembly is used for driving the susceptor (1) to generate heat.
